# FASCICULE DE BREVET EUROPEEN

(11) **EP 2 396 319 B1**
(45) Date de publication et mention de la délivrance du brevet: **17.07.2013**
(21) Numéro de dépôt: 10708306.5
(22) Date de dépôt: 09.02.2010
(51) Int. Cl.: C07D 407/04, A61K 31/496, A61P 3/00

(54) **DERIVES DE 3-BENZOFURANYL-INDOL-2-ONE-3-ACETAMIDOPIPERAZINES SUBSTITUES, LEUR PREPARATION ET LEUR APPLICATION EN THERAPEUTIQUE**
SUBSTITUIERTE 3-BENZOFURANYLINDOL-2-ON-3-ACETAMIDO-PIPERAZINDERIVATE, DEREN HERSTELLUNG UND DEREN THERAPEUTISCHE VERWENDUNG
SUBSTITUTED 3-BENZOFURANYL-INDOL-2-ONE-3-ACETAMIDOPIPERAZINE DERIVATIVES, PREPARATION THEREOF, AND THERAPEUTIC USE THEREOF

(30) Priorité: 12.02.2009 FR 0900620
(43) Date de publication de la demande: 21.12.2011
(73) Titulaire: SANOFI, 75008 Paris (FR)
(72) Inventeur: BARONI, Marco, F-75013 Paris (FR); PULEO, Letizia, F-75013 Paris (FR)
(74) Mandataire: Veinante, Aude
(86) Numéro de dépôt international: PCT/FR2010/050206
(87) Numéro de publication internationale: WO 2010/092288

(56) Documents cités:
- WO-A-2005/035498
- WO-A-2009/056707

## Description

La présente invention a pour objet des dérivés de 3-benzofuranyl-indol-2-one-3-acétamidopipérazines substitués, leur préparation et leur application en thérapeutique.

La ghréline est une hormone peptidique de 28 acides aminés produite de façon principale dans l'estomac par un processus post-traductionnel après clivage de la pré-pro-ghréline (Kojima M, et al., Nature 1999; 402:656-60). La ghréline est un ligand endogène du récepteur hypophysaire des sécrétagogues de l'hormone de croissance (GHSR1a).

GHS-R est codé par deux exons : l'exon 1 code pour les domaines transmembranaires (TMs) 1-5 et l'exon 2 code pour TM6 et 7 du récepteur couplé à la protéine G (GPCR).

Les deux transcripts ont été identifiés dans la glande pituitaire et le cerveau : l'un codant pour le GPCR de longueur totale (GHS-R1a) et l'autre codant pour un récepteur tronqué (GHS-R1b) auquel manquent TM6 et 7. Seul le sous-type GHS-R1a est activé par la ghréline et les mimétiques de la ghréline. GHS-R1 b est présent dans le foie et d'autres tissus périphériques mais sa fonction est inconnue (Smith R.G. et al, Trends in Endocrinology and Metabolism, 2005, 16 No. 9).

C'est un récepteur de type rhodopsine, à sept domaines transmembranaires de la famille A couplé à Gq/phospholipase C. Le récepteur de la ghréline peut aussi être couplé aux voies Gs/protéine kinase A dans certains tissus (Ueno, N. et al. Endocrinology, 2004, 145, 4176-4184 ; Kim, M.S. et al., Int. J. Obes. Relat. Metab. Disord., 2004, 28:1264-1271). De façon intéressante, le récepteur de la ghréline présente la caractéristique peu commune de présenter une activité constitutive indépendante du ligand significative (Barazzoni, R. et al., Am. J. Physiol. Endocrinol. Metab., 2004, 288: E228-E235).

Des niveaux faibles de l'expression de la ghréline ont été documentés dans des tissus variés, tels que les intestins, le pancréas, les reins, le système immunitaire, le placenta, les testicules, les tissus pituitaires, l'hypothalamus (Horm. Res. 2003; 59 (3):109-17).

Il a été démontré que la ghréline est impliquée dans la faim au moment des repas, et dans l'initiation des repas. Les niveaux circulants diminuent avec la prise d'alimentation et augmentent avant les repas, atteignant des concentrations suffisantes pour stimuler la faim et la prise de nourriture. Les ingestions de ghréline stimulent la prise de nourriture rapidement et de façon transitoire, principalement en augmentant les comportements d'alimentation appétante et le nombre de repas. La ghréline stimule la prise de nourriture à court terme de façon plus efficace que n'importe quelle autre molécule, à l'exception du neuropeptide Y, avec lequel elle est approximativement équipotente (Wren A.M., et al., J. Clin. Endocrinol. Metab., 2001;86:5992-5.). Cependant, la ghréline est unique dans sa capacité à exercer cet effet, qu'elle soit injectée de façon périphérique ou centrale.

C'est également la seule substance de mammifère qui a démontré sa capacité à augmenter l'appétit et la prise d'alimentation lorsqu'elle est administrée aux humains (Druce M.R., et al., Int. J. Obes., 2005; 29:1130-6; Wynne K., et al., J. Am. Soc. Nephrol., 2005; 16: 2111-8.).

Au-delà de son rôle dans l'initiation des repas, la ghréline remplit également les critères établis d'une hormone liée à l'adiposité impliquée dans la régulation de la masse corporelle à long terme. Les taux de ghréline circulent en fonction des stocks énergétiques et manifestent des changements compensatoires en réponse aux modifications de la masse corporelle.

La ghréline traverse la barrière hémato-encéphalique et stimule la prise de nourriture en agissant sur certains centres régulateurs classiques de la masse corporelle, tels que l'hypothalamus, le cerveau postérieur et le système compensatoire mésolimbique.

L'administration chronique de ghréline augmente la masse corporelle par diverses actions concertées sur la prise de nourriture, la dépense énergétique et l'utilisation des ressources. L'ablation congénitale de la ghréline ou du gène du récepteur de la ghréline provoque une résistance à l'obésité induite par l'alimentation, et le blocage pharmacologique de la ghréline réduit la prise de nourriture et la masse corporelle.

Les preuves existantes semblent favoriser le rôle de la ghréline à la fois dans l'initiation des repas à court terme et l'homéostasie énergétique à long terme, en faisant ainsi une cible attractive comme médicament pour traiter l'obésité et/ou les désordres de l'amaigrissement.

La ghréline exerce également des actions à la fois physiologiques et pharmacologiques sur le pancréas endocrine. La ghréline bioactive acylée est produite dans la cellule-ε, récemment décrite dans les îlots pancréatiques (Prado, C.L., et al., 2004, Proc. Natl. Acad. Sci. USA, 101:2924-2929), fournissant potentiellement une source locale de ghréline qui agit sur les cellules β des îlots. Le blocage de cette fonction de la ghréline endogène par le biais d'un antagoniste pour ses récepteurs a diminué les concentrations de glucose à jeûn de façon prononcée, a atténué le mouvement glycémique et augmenté les réponses à l'insuline lors des tests à la tolérance au glucose, suggérant un rôle inhibiteur pour la ghréline dans le contrôle de la sécrétion de l'insuline (Dezaki, K., et al. 2004, Diabètes, 53:3142-3151).

L'ablation de la ghréline chez les souris (souris ghréline -/-) augmente la sécrétion d'insuline dépendante du glucose par la cellule β du pancréas par réduction de l'expression Ucp2 et améliore la sensibilité à l'insuline périphérique (Sun Y. et al., 2006, Cell Metabolism, 3:379-386).

Des antagonistes du récepteur de la ghréline pourraient ainsi réguler la faim, la prise de repas et leur fréquence, ainsi que, à long terme, le poids, notamment la prise de poids faisant suite aux régimes diététiques ou thérapeutiques. De plus, dans le cadre d'un traitement antidiabétique, les antagonistes de la ghréline pourraient être utiles pour maintenir l'équilibre entre insuline et glucose et pour contrôler l'hyperphagie diabétique. Les antagonistes de la ghréline pourraient ainsi être utilisés comme agents anorexiques et/ou anti-obésité, ou encore dans le traitement du diabète et de ses effets.

La présente invention a pour objet les composés répondant à la formule (I) : 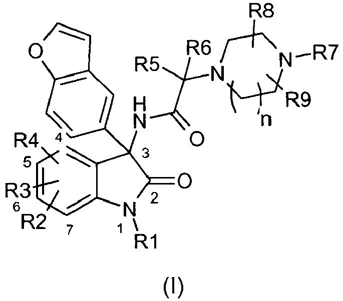 dans laquelle :
R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy , hétéroaryle ; le groupe aryle, aryloxy ou hétéroaryle pouvant éventuellement être substitué par un atome d'halogène, CN, OH ou un groupe (C1-6) alkyle, perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle, perhalogéno(C1-3) alkyle, (C1-6) alcoxy;
R5 et R6, identiques ou différents, représentent un atome d'hydrogène, un groupe (C1-6) alkyle ou R5 et R6 forment ensemble un cycle C3-C6 ;
R7 représente un groupe (C1-C6) alkyle ou un groupe (C2-6) alcényle ;
R8 et R9, situés sur l'une quelconque des positions disponibles du noyau de la pipérazine, représentent un atome d'hydrogène, un groupe (C1-C6) alkyle ou un groupe (C2-6) alcényle, étant entendu que au moins un des R8 et R9 est différent de H ;
ou deux des R7, R8 et R9 forment ensemble un cycle C3-C6 ;
étant entendu que R8 et R9 peuvent se trouver en position géminale sur le même atome de carbone ;
n représente 1 ou 2.

Les composés de formule (I) comportent un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par :
- un atome d'halogène : un fluor, un chlore, un brome ou un iode ;
- un groupe alkyle : un groupe aliphatique saturé linéaire ou ramifié. A titre d'exemples, on peut citer un groupe (C1-6) alkyle comprenant de 1 à 6 atomes de carbone, plus particulièrement (C1-4) alkyle qui peut représenter un méthyle, éthyle, propyle, isopropyle, butyle, isobutyle, tertbutyle ;
- un groupe alcényle : un groupe aliphatique mono- ou poly-insaturé, linéaire ou ramifié, comprenant par exemple une ou deux insaturations et comprenant de 2 à 6 atomes de carbone ;
- un groupe halogénoalkyle un groupe alkyle dont un ou plusieurs atomes d'hydrogène ont été substitués par un atome d'halogène ; par exemple un fluoroalkyle : un groupe alkyle dont un ou plusieurs atomes d'hydrogène ont été substitués par un atome de fluor ;
- un groupe perhalogénoalklyle : un groupe alkyle dont tous les atomes d'hydrogène ont été substitués par un atome d'halogène ; par exemple un perfluoroalkyle : un groupe alkyle dont tous les atomes d'hydrogène ont été substitués par un atome de fluor ;
- un groupe alcoxy : un radical -O-alkyle où le groupe alkyle est tel que précédemment défini;
- un groupe perhalogénoalcoxy : un radical -O-perhalogénoalkyle où le groupe perhalogénoalkyle est tel que précédemment défini, à titre d'exemple on peut citer le trifluorométhoxy;
- un groupe aryle : un groupe aromatique cyclique comprenant entre 6 et 10 atomes de carbone. A titre d'exemples de groupes aryles, on peut citer un phényle ou naphthyle;
- un groupe hétéroaryle : un groupe aromatique cyclique comprenant entre 2 et 10 atomes de carbone et comprenant entre 1 et 3 hétéroatomes, tels que l'azote, l'oxygène ou le soufre. A titre d'exemples de groupes hétéroaryles, on peut citer les groupes furanyle, pyrrolyle, imidazolyle, pyrazolyle, thiényle, oxadiazolyle, oxazolyle, isoxazolyle, furazanyle, thiadiazolyle, thiazolyle, isothiazolyle, pyridyle, pyrazinyle, pyrimidinyle, pyridazinyle ainsi que les groupes correspondants résultants de la fusion avec un groupe phényle tel que par exemple benzothiophène, benzofurane; benzothiazole, ...

Parmi les composés de formule (I) objets de l'invention, un groupe de composés est constitué par les composés pour lesquels :
R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle, perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy , hétéroaryle, étant entendu que au moins un des R2, R3, R4 est différent de H ;
R5 et R6, identiques ou différents, représentent un atome d'hydrogène, un groupe (C1-6) alkyle ou R5 et R6 forment ensemble un cycle C3-C6;
R7 représente un groupe (C1-C6) alkyle;
R8 et R9, situés sur l'une quelconque des positions disponibles du noyau de la pipérazine, représentent un atome d'hydrogène ou un groupe (C1-C6) alkyle, étant entendu que au moins un des R8 et R9 est différent de H ;
ou deux des R7, R8 et R9 forment ensemble un cycle C3-C6 ;
n représente 1 ou 2 ;
sous forme de base ou de sel d'addition à un acide.

Parmi les composés de formule (I) objets de l'invention, un groupe de composés est constitué par les composés pour lesquels :
R1 représente un atome d'hydrogène ou un groupe -C(=O)(C1-6) alkyle, -C(=O)aryle, (C1-6) alkyle ; et/ou
R2, R3, R4, identiques ou différents, situés sur l'une quelconque des positions disponibles du noyau phényle, représentent indépendamment un atome d'hydrogène, un atome d'halogène, plus particulièrement le chlore ou le brome, ou un groupe (C1-6) alkyle ou trifluorométhyle, étant entendu que au moins un des R2, R3, R4 est différent de H ; et/ou
R5 et R6, identiques ou différents, représentent un atome d'hydrogène ou un groupe (C1-6) alkyle ; et/ou
R7 représente un groupe (C1-C6) alkyle; et/ou
R8 et R9, situés sur les positions 2 et 6 du noyau de la pipérazine, représentent un atome d'hydrogène ou un groupe (C1-C6) alkyle, étant entendu que au moins un des R8 et R9 est différent de H ; et/ou
deux des R7, R8 et R9 forment ensemble un cycle C3-C6; et/ou
n représente 1 ou 2 ;
sous forme de base ou de sel d'addition à un acide.

Parmi les composés de formule (I) objets de l'invention, un autre groupe de composés est constitué par les composés pour lesquels :
R1 représente un atome d'hydrogène ou un groupe -C(=O)méthyle, -C(=O)phényle, méthyle ; et/ou
R2, R3, R4, identiques ou différents, situés sur l'une quelconque des positions disponibles du noyau phényle, représentent indépendamment un atome d'hydrogène, un atome d'halogène, plus particulièrement le chlore ou le brome, ou un groupe méthyle ou trifluorométhyle, étant entendu que au moins un des R2, R3, R4 est différent de H ; et/ou
R5 et R6, identiques ou différents, représentent un atome d'hydrogène ou un groupe (C1-6) alkyle ; et/ou
R7 représente un groupe méthyle ou éthyle; et/ou
R8 et R9, situés sur les positions 2 et 6 du noyau de la pipérazine, représentent un atome d'hydrogène ou un groupe méthyle, un groupe éthyle, étant entendu que au moins un des R8 et R9 est différent de H ; et/ou
deux des R7, R8 et R9 forment ensemble un cycle C3-C6 ; et/ou
n représente 1 ou 2 ;
sous forme de base ou de sel d'addition à un acide.

Parmi les composés de formule (I) objets de l'invention, on peut notamment citer les composés suivants :
Composé n°1 : (+) N-[4,6-Dichloro-3-(benzofuran-5-yl)-2-oxo-2,3-dihydro-1H-indol-3-yl]-2-(4-éthyl-3,5-diméthyl-pipérazin-1-yl)-acétamide ;
Composé n°2 : (+) N-[4,6-Dichloro-3-(benzofuran-5-yl)-2-oxo-2,3-dihydro-1H-indol-3-yl]-2-(4-éthyl-pipérazin-1-yl)-propionamide ;
sous forme de base ou de sel d'addition à un acide.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans Protective Groups in Organic Synthesis, Green et al. 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans Advances in Organic Chemistry, J. March, 3rd Edition, Wiley Interscience, p. 310-316.

Conformément à l'invention, on peut préparer les composés de formule générale (I) selon le procédé qui suit : 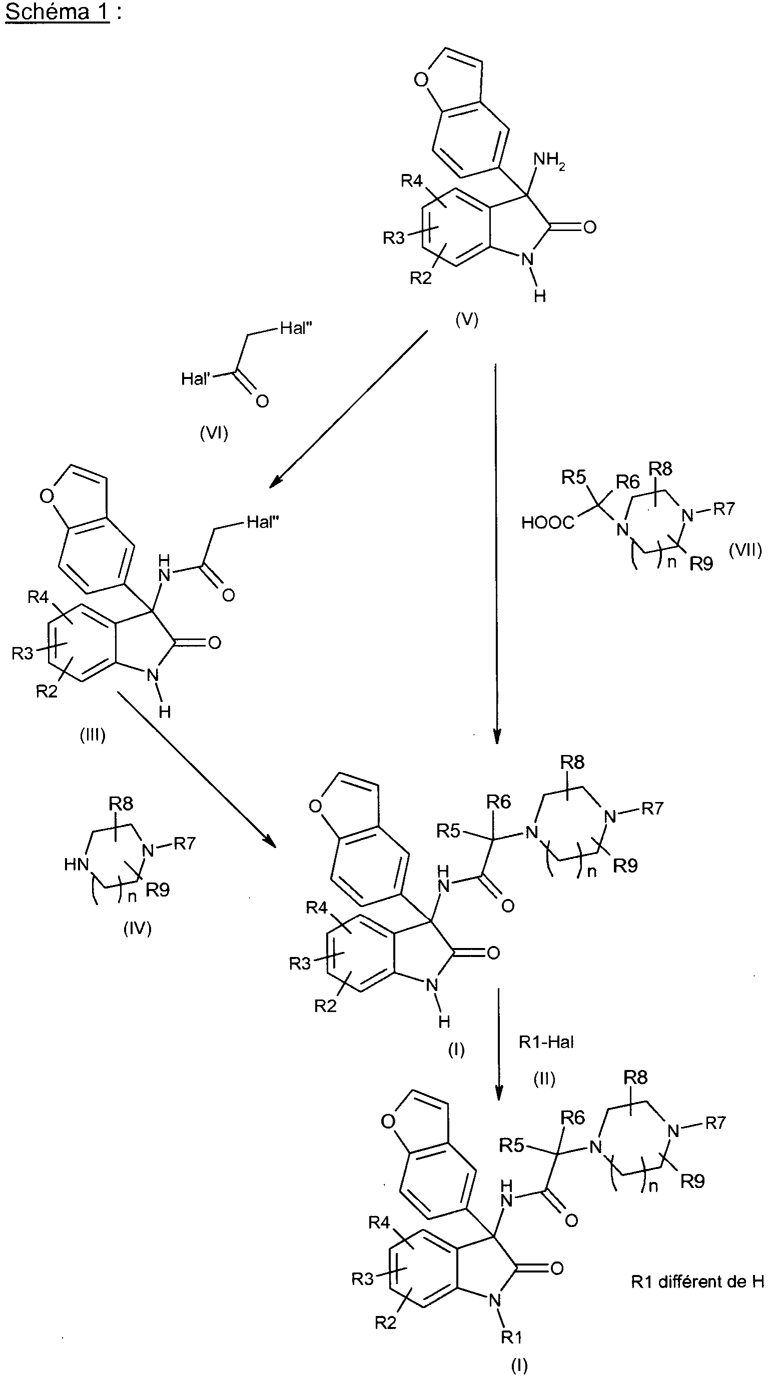 Le composé de formule (I), dans laquelle R1 est différent de H, R2, R3, R4, R5, R6, R7, R8, R9 et n sont tels que définis en formule générale (I) peut être préparé par réaction d'un composé de formule (I) dans laquelle R1=H avec un composé de formule (II) :

R1-Hal (II)

dans laquelle R1, différent de H est défini comme en formule générale (I) et Hal représente un atome d'halogène, par exemple le chlore selon des méthodes connues de l'homme du métier par exemple en présence d'une base, telle que K₂CO₃, NaH, t-BuO⁻K⁺, dans un solvant tel que le diméthylformamide (DMF), tétrahydrofurane (THF), dimethoxyéthane, le diméthylsulfoxide (DMSO).

Le composé de formule générale (I) peut être préparé selon l'une des variantes suivantes :

Le composé de formule générale (I) dans laquelle R1 =H peut être préparé selon la méthode suivante, à partir d'un composé de formule générale (V) : 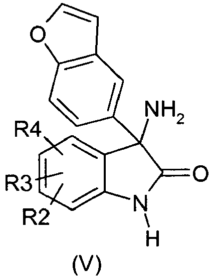 et d'un composé de formule générale (VII) : 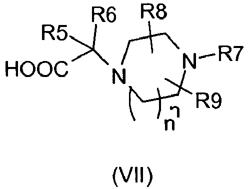 dans lesquelles, R2, R3, R4, R5, R6, R7, R8, R9 et n sont tels que définis en formule générale (I). Cette réaction est généralement effectuée au moyen d'un agent d'halogénation, tel qu'un agent de chloration, par exemple les chlorures de phosphore, notamment PCl₅, ou encore PCl₃ ou POCl₃. La réaction est généralement réalisée en présence de pyridine ou 4-diméthylaminopyridine, dans un solvant tel que le dichlorométhane ou le DMF.

Le composé formule générale (I) dans laquelle R1, R5 et R6 =H peut être préparé selon la méthode suivante, par réaction d'un composé de formule générale (III) : 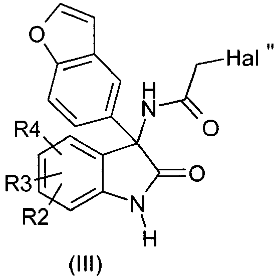 avec un composé de formule générale (IV) : 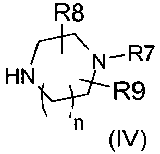 dans lesquelles, R2, R3, R4, R7, R8, R9 et n sont tels que définis en formule générale (I) et Hal" représente un atome d'halogène, de préférence le chlore. Cette réaction est généralement effectuée au moyen d'une base, organique ou minérale, telle que K₂CO₃, Na₂CO₃, la pyridine ou la 4-diméthylaminopyridine, en présence de Nal ou KI, dans un solvant inerte tel que le DMF, le dichlorométhane, le THF, le diméthoxyéthane ou le toluène.

Le composé de formule générale (III) peut être préparé à partir d'un composé de formule générale (V) : 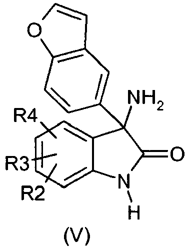 et d'un composé de formule générale (VI) : 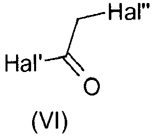 dans lesquelles R2, R3, R4, sont tels que définis en formule générale (I) et Hal' et Hal", identiques ou différents représentent indépendamment un atome d'halogène, de préférence le chlore.

Cette réaction est généralement effectuée au moyen de pyridine ou 4-diméthylaminopyridine dans un solvant tel que le toluène, benzène ou dichlorométhane, préférentiellement à température comprise entre la température ambiante et la température de reflux du solvant.

Par température ambiante, on entend une température comprise entre 5 et 25°C.

Les intermédiaires de formule générale (V) sont connus et peuvent être préparés selon les procédés illustrés par le schéma qui suit : 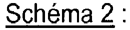 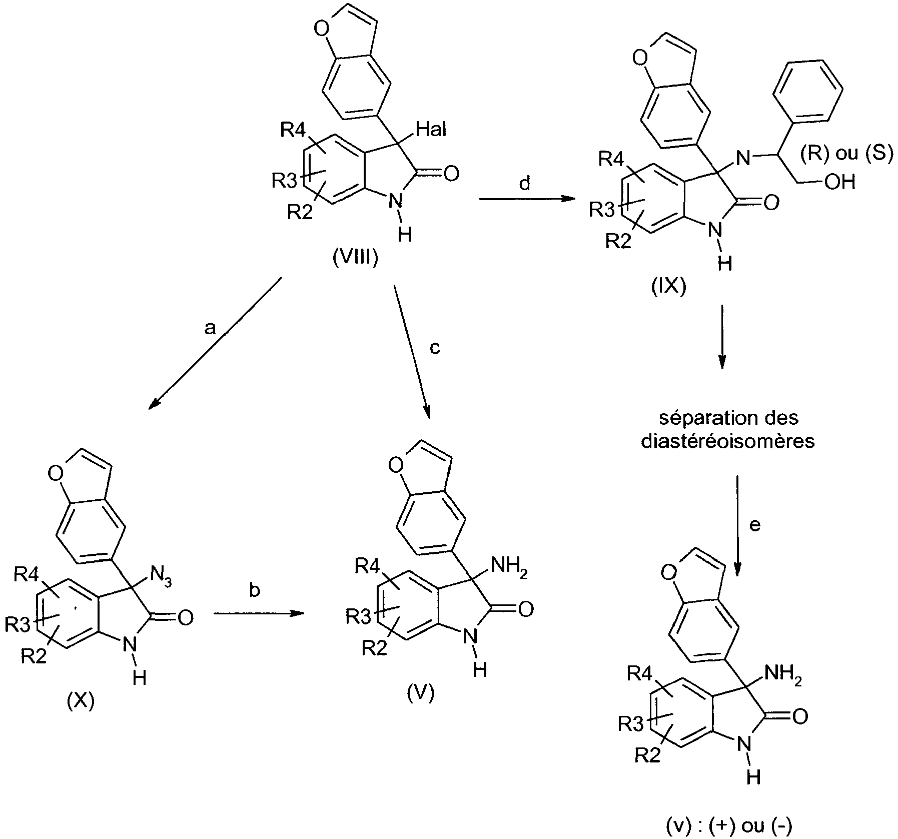 dans lequel R2, R3 et R4 sont tels que définis en formule générale (I) et Hal représente un atome d'halogène, par exemple le chlore.

A l'étape c du Schéma 2, on prépare un composé de formule (V) à partir d'un composé de formule (VIII) par barbotage d'ammoniac gazeux selon la méthode décrite dans la demande FR 2 714 378.

On peut également préparer le même composé par réduction d'un composé de formule (X) selon des méthodes connues de l'homme du métier, par exemple au moyen du zinc dans un solvant tel que le méthanol. La préparation d'un composé de formule (X) de l'étape est décrite dans la demande FR 2 714 378.

Un composé de formule (V) optiquement pur peut être synthétisé selon les étapes d et e du Schéma 2, telles que décrites dans la demande WO 03/008407.

Les intermédiaires de formule générale (VIII) peuvent être préparés selon le procédé décrit dans la demande WO 03/008407 et illustrés par le schéma 3 : 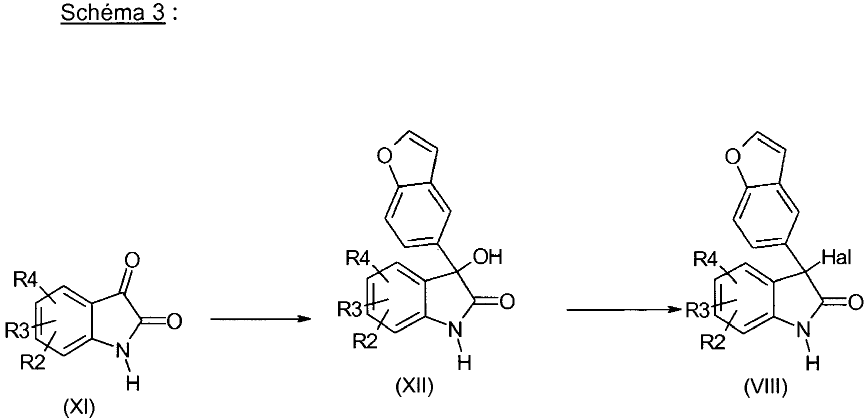 dans lequel R2, R3 et R4 sont tels que définis en formule générale (I) et Hal représente un atome d'halogène, par exemple le chlore.

Le composé de formule générale (VII) peut être préparé selon les méthodes suivantes, illustrées par le schéma 4 et 5 :

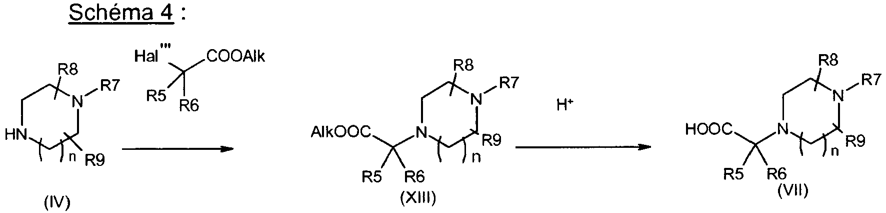

Le composé de formule générale (XIII) peut être préparé par condensation d'un composé de formule générale (IV) : 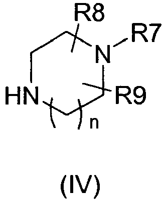 dans laquelle R7, R8, R9 et n sont définis comme en formule générale (I), avec un composé halogéné correspondant, tel que Hal"'CH₂COOAlk, où Hal"' représente un atome d'halogène, tel que le chlore et Alk représente un groupe alkyle, tel que éthyle. Cette réaction est avantageusement effectuée dans un solvant tel que le toluène, le benzène ou le dioxane.

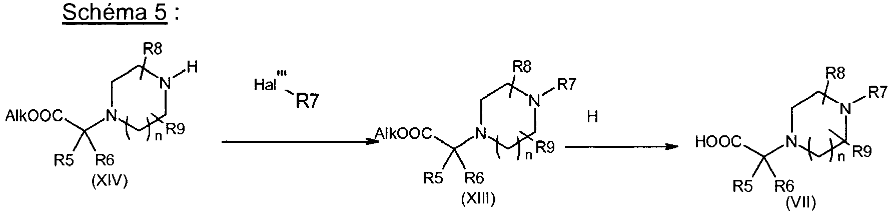

Le composé de formule générale (XIII) peut être préparé par condensation d'un composé de formule générale (XIV) : 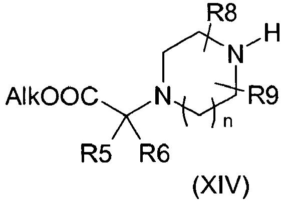 dans laquelle R5, R6, R8, R9 et n sont définis comme en formule générale (I) et Alk représente un groupe alkyl, avec un composé R7-Hal"' ou Hal"' représente un atome d'halogène, tel que le chlore et R7 défini comme en formule générale (I). Cette réaction est avantageusement effectuée dans un solvant tel que le toluène, le benzène, le dioxane ou le DMF en présence d'une base tel que la triethylamine ou le carbonate de potassium.

Selon un autre mode de réalisation les composés de formule générale (I) dans laquelle R1 représente un group alkyle et R2, R3, R4, R5, R6, R7,R8, R9 et n sont tels que définis en formule générale (I) peuvent également être préparés selon le schéma 6 suivant :

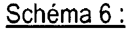

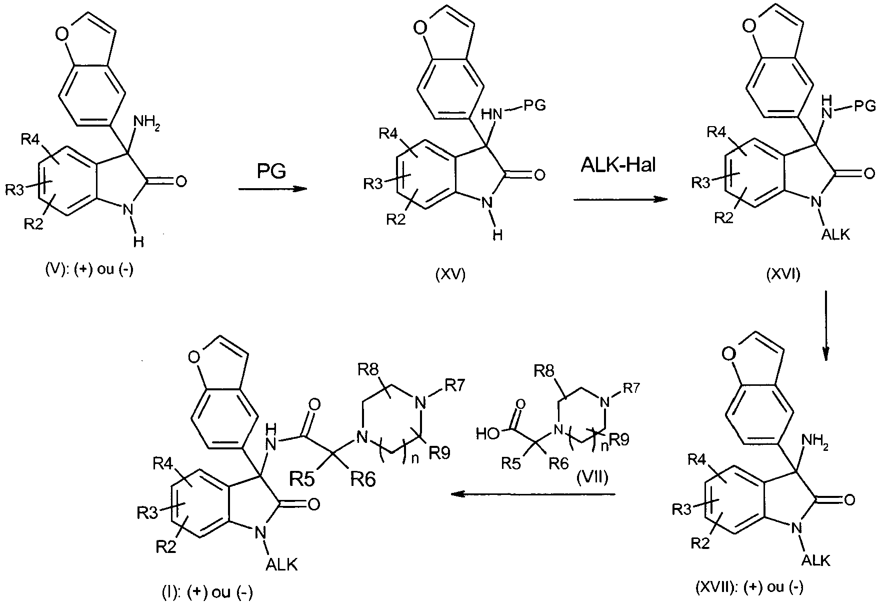

Selon ce schéma, on fait réagir un composé de formule (V) avec un groupe protecteur PG pour donner le composé de formule (XV). En tant que groupe protecteur PG de l'amine, on peut utiliser par exemple la benzimine ou le t-butyl carbamate. Ces derniers sont introduits selon des méthodes connues de l'homme du métier par exemple en présence d'une base, telle que K₂CO₃, NaOH, triéthylamine dans un solvant tel que le dioxane, THF ou le DMSO.

Le composé de formule générale (XVI) peut être préparé par réaction d'un composé de formule (XV) avec un composé ALK-Hal, dans laquelle ALK représente un groupe aliphatique saturé linéaire ou ramifié comprenant de 1 à 6 atomes de carbone et Hal représente un atome d'halogène, par exemple le chlore.

On obtient le composé de formule générale (XVII) à partir d'un composé de formule (XVI) par élimination du groupe protecteur selon des méthodes bien connues, par exemple en milieu acide par HCl ou acide trifluoroacétique.

On fait réagir ensuite le composé de formule (XVII) obtenu avec un composé de formule générale (VII) : 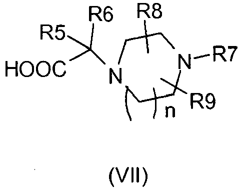 dans lesquelles, R2, R3, R4, R5, R6, R7, R8, R9 et n sont tels que définis en formule générale (I). Cette réaction est généralement effectuée au moyen d'un agent d'halogénation, tel qu'un agent de chloration, par exemple les chlorures de phosphore, notamment PCl₅, ou encore PCl₃ ou POCl₃. La réaction est généralement réalisée en présence de pyridine ou 4-diméthylaminopyridine, dans un solvant tel que le dichlorométhane ou le DMF.

Eventuellement, on transforme le composé de formule (I) en l'un de ses sels d'addition à un acide.

Le procédé selon l'invention peut éventuellement comprendre l'étape consistant à isoler le produit de formule générale (I) désiré.

Dans les schémas 1, 2, 3, 4, 5 et 6, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les exemples suivants décrivent la préparation de certains composés conformes à l'invention. Ces exemples ne sont pas limitatifs et ne font qu'illustrer la présente invention.

Les mesures physico-chimiques ont été effectuées de la façon suivante : Les points de fusion ont été mesurés avec un appareil BUCHI B-540.

Les spectres de résonance magnétique nucléaire du proton (RMN 1 H) ont été enregistrés à 500 MHz sur un appareil Bruker équipé avec une console Avance III. Les déplacements chimiques sont rapportés en ppm par rapport à la fréquence du TMS.

Touts les spectres ont été enregistrés à la température de 40°C.

Les abréviations utilisées pour caractériser les signaux sont les suivantes: s = singulet, bs =singulet large, m = multiplet, bm= multiplet large, d = doublet, bd= doublet large, t = triplet, q = quadruplet.
* = non intégrable à cause de l'interférence avec un pic large du à l'eau.
**= non intégrable à cause de l'interférence avec un pic du au solvant RMN.
***= lu au premier ordre.
****= diastéréoisomère le plus abondant.
*****= diastéréoisomère le moins abondant.

Les conditions d'analyse par chromatographie liquide couplée à la spectrométrie de masse (LC/UV/MS) sont les suivantes:
Pour la partie chromatographie liquide
Colonne XTerra MS C18 3,5 µm
   - système chromatographique
   - Eluant A = H₂O + 0,01% TFA
   - Eluant B = CH₃CN.
   - Gradient de 98% de A à 95% de B en 10 minutes, puis élution par 95% de B pendant 5 minutes.
   - Débit 0,5ml/minute
   - Injection de 2µL de solution à 0,1mg/ml dans un mélange CH₃CN : H₂O = 9 :1

Les produits sont détectés en UV à 220 nm.

Pour la partie spectrométrie de masse :
- Mode d'ionisation: électrospray positif (API-ES polarité+)
- Balayage de 100 à 1200 uma

La chromatographie en couche mince a été effectuée sur des plaques CCM de gel de silice de Merck. Le gel de silice pour la chromatographie sur colonne flash est commercialisé par Biotage.

Tous les solvants utilisés sont de pureté "reagent grade" ou "HPLC grade".

Les mesures de Alpha D ont été effectuées sur un polarimétre Perkin Elmer modéle PE341 en utlilisant une cellule avec un chemin optique de 1 dm.

Dans les exemples et préparations :
AcOH et AcOEt représentent respectivement l'acide acétique et l'acétate d'éthyle.
MeOH, EtOH, tBuOH représentent respectivement méthanol, éthanol et ter-butanol.
THF représente le Tétrahydrofurane
Pf représente le point de fusion.

### Préparation 1:

### Acide 2- (4-éthyl-pipérazin-1-yl) propionique

### (i) éthyle 2- (4-éthyl-pipérazin-1-yl)-propionate

Dans un ballon, on charge 9,7 g d'éthyl 2-pipérazin-1-yl propionate dans 150 ml de DMF et 21,6 g de carbonate de potassium. On ajoute goutte à goutte une solution de 3,9 ml de bromoéthane. On laisse réagir à 130°C pendant trois heures, on filtre le carbonate et on concentre à sec. On reprend avec de l'acétate d'éthyle. On filtre. Le solide est éliminé, la phase liquide est évaporée sous vide. On obtient un solide cristallisé avec de l'acétate d'éthyle. On filtre et on obtient 8,46 g du produit du titre.
CCM : MeOH 100%, Rf=0,55

### (ii) Acide 2-(4-éthyl-pipérazin-1-yl) propionique

On ajoute 8, 45 g du produit obtenu à l'étape précédente à 180 ml de HCl 6N et on laisse réagir 4 heures à reflux. On évapore à sec, on lave avec un mélange AcOEt 1 / EtOH 1 et on sèche le solide blanc obtenu. On obtient 5,4 g de produit attendu.
CCM: 100% MeOH, Rf=0,2

### Préparation 2:

### (+) 3-Amino-4,6-dichloro-1,3-dihydro-3-(benzofuran-5-yl)-indole-2-one

### (i) 3-Hydroxy-4,6-dichloro-1,3-dihydro-3-(benzofuran-5-yl)-indole-2-one

Dans un ballon muni d'un agitateur mécanique et sous flux d'azote, on charge 2,25 g de magnésium pour Grignard dans 15 ml de THF anhydre. Puis on ajoute un mélange de 13,6 g de 5-bromobenzofurane dans 35 ml de THF anhydre. On laisse sous agitation pendant une heure, puis on ajoute une solution de 5 g de 4,6-dichloro-1H-indol-2,3-dione dans 50 ml de THF anhydre. On laisse agiter à température ambiante pendant 4 heures et demie. On ajoute de l'eau, et on extrait avec de l'acétate d'éthyle. On sépare la phase organique que l'on sèche sur Na₂SO₄, on filtre et on évapore sous vide. On reprend avec de l'acétate d'éthyle et on lave avec une solution de soude 1 N. La phase organique est séchée sur Na₂SO₄, filtrée et évaporée sous vide. Le solide est repris avec de l'éther éthylique et filtré. On obtient 4,2 g de produit attendu.

### (ii) 3,4,6-trichloro-1,3-dihydro-3-(benzofuran-5-yl)-indole-2-one

Dans un ballon muni d'un agitateur magnétique et sous flux d'azote, on charge 4,1 g du produit de l'étape précédente, dans 40 ml de dichlorométhane. A 0°C, on ajoute 1,7 ml de pyridine et un mélange de 1,4 ml de SOCl₂ dans 30 ml de dichlorométhane. On laisse réagir à température ambiante, puis on verse dans une solution aqueuse saturée de NH₄Cl. On sépare la phase organique qui est séchée sur Na₂SO₄, filtrée et évaporée sous vide.
CCM: Hex.7/ AcOEt 3, Rf=0,65

### (iii) 4,6-dichloro-[[(1S)-2-hydroxy-1-phényléthyl] amino] 1,3-dihydro -3-(benzofuran-5-yl)-indole-2-one, isomère A et isomère B

Sous flux d'azote, on mélange 4,1 g du composé de l'étape précédente dans 50 ml de dichlorométhane et 3,1 g de S-phénylglycynol. On laisse réagir une nuit à température ambiante. On filtre le solide formé, les eaux de filtration sont évaporées à sec et purifiées sur colonne avec l'éluant Hex. /AcOEt 8:2.

On obtient 0, 64 g de produit moins polaire, isomère A (pf=135°C) et 1,23 g de l'isomère plus polaire B.

### (iv) (+)-3-Amino-5,6-dichloro-1,3-dihydro-3-(4-chlorophényl)-indole-2-one

On met à réagir 1,21 g du produit obtenu dans l'étape précédente dans un mélange de 20 ml de dichlorométhane et 15 ml de méthanol. On ajoute 1,26 g de Pb(OAc)₄ et on laisse réagir à température ambiante pendant 1 heure. On évapore à sec et on reprend avec de l'acétate d'éthyle puis on lave avec une solution aqueuse saturée de NaHCO₃. La phase organique est séchée, filtrée et concentrée. On reprend avec un mélange de 36 ml d'acide chlorhydrique 3N et 3,7 ml de méthanol et on laisse sous agitation pendant une nuit. On concentre et on dilue avec un mélange d'eau et de dichlorométhane. La phase organique est lavée avec une solution d'acide chlorhydrique 1 N. Les phases aqueuses sont réunies, portées à pH basique avec une solution de NH₃ aqueuse, et extraites avec du dichlorométhane. La phase organique est séchée, filtrée et concentrée pour obtenir 870 mg de produit solide blanc.
Pf: 215-216°C
LC/MS : (M+H)⁺ = m/z 333 uma; rt = 5,3 minutes

### Exemple 1

### (+) N-[4,6-Dichloro-3-(benzofuran-5-yl)-2-oxo-2,3-dihydro-1H-indol-3-yl]-2-(4-éthyl-3,5-diméthyl-pipérazin-1-yl)-acétamide

### (i) 2-Chloro-N-[4,6-dichloro-3-(benzofuran-5-yl)-2-oxo-2,3-dihydro-1H-indol-3-yl]-acétamide :

Dans un ballon muni d'un agitateur magnétique et sous flux d'azote, on charge 0,43 g du produit obtenu à la Préparation 2, 15 ml de toluène, 0,11 ml de pyridine et 0,11 ml de chlorure de chloroacétyle. On laisse réagir à 110°C pendant 4 heures, puis on verse le mélange réactionnel dans l'eau et on extrait à l'acétate d'éthyle. La phase organique est séchée sur Na₂SO₄, filtrée et évaporée sous vide. On obtient 500 mg de solide beige, purifié sur colonne par flash chromatographie au moyen du mélange cyclohexane 8/ acétate d'éthyle 2 pour obtenir 330 mg du produit attendu.
CCM: Hex. 1/ AcOEt 1, Rf=0,5

### (ii) (+) N-[4,6-Dichloro-3-(benzofuran-2-yl)-2-oxo-2,3-dihydro-1H-indol-3-yl]-2-(3,5-diméthyl-4-éthyl-pipérazin-1-yl)-acétamide:

Dans un ballon muni d'un agitateur magnétique, on charge 0,31 g du produit de l'étape précédente, 0,08 ml de 2,6-diméthyl-N-éthylpipérazine (d 0,899), 0,1 g de carbonate de potassium, 0,05 g de iodure de sodium dans 5 ml de DMF. On laisse réagir à 60°C pendant 4 heures puis on verse le mélange réactionnel dans l'eau et on extrait à l'acétate d'éthyle. La phase organique est séchée sur Na₂SO₄, filtrée et évaporée sous vide. On obtient le produit du titre
P.f. = 157-160°C; [α_{D}]= + 191°, c=0,946 wt% MeOH ;
LC/MS : (M+H)⁺ = m/z 515 uma; rt = 4,9 minutes

### Exemple 2

### (+) N-[4,6-Dichloro-3-(benzofuran-5-yl)-2-oxo-2,3-dihydro-1H-indol-3-yl]-2-(4-éthyl-pipérazin-1-yl)-propionamide

1) Sous flux d'azote, on ajoute dans 12 ml de dichlorométhane anhydre refroidi dans un bain de glace, 520 mg de PCl₅ puis on ajoute lentement 430 mg de l'acide de la Préparation 1. On laisse agir le mélange réactionnel à 0°C pendant 10 minutes puis à température ambiante pendant 3 heures.
2) D'autre part, on suspend sous flux d'azote 300 mg du produit de la préparation 2, dans 12 ml de dichlorométhane puis on ajoute 0,3 ml de pyridine. On refroidit dans un bain de glace. On ajoute goutte à goutte la solution préparée en 1) et on agite à température ambiante pendant une heure.

On verse le mélange réactionnel dans l'eau et on extrait à l'acétate d'éthyle. La phase organique est lavée avec une solution saturée de NaHCO₃, séchée sur Na₂SO₄, filtrée et évaporée sous vide. On obtient 500 mg de solide orange, purifié sur colonne par flash chromatographie au moyen de l'éluant acétate d'éthyle 1 / méthanol 1 pour obtenir le produit du titre
P.f. =137-138°C; [α_{D}]= +217°, c=0,1064 wt% in MeOH,

RMN : δ (ppm, dmso-d6): 0,99 (m, 3H), 1,08 (m, 3H), 2,24 - 2,45 (m, 6H), 2,47 - 2,68 (m, **), 3,18 - 3,33 (m, *), 6,92 (m, 1 H), 7,01 (s, 1 H), 7,19 (m, 1H), 7,21 - 7,28 (m, 1 H), 7,50 (bs, 1 H), 7,65 (d, J= 8.8Hz, 1 H), 8,02 (s, 1 H), 8,61 (s, 0.4H*****), 8,73 (s, 0.6H****), 10,64 (s, 0.6H****), 10,71 (s, 0.4H*****).
LC/MS : (M+H)⁺ = m/z 501 uma; rt = 4,9 minutes

### Exemple 3

### (+) N-[4,6-Dichloro-3-(benzofuran-5-yl)-2-oxo-2,3-dihydro-1H-indol-3-yl]-2-(4-éthyl-3-méthyl-pipérazin-1-yl)-acétamide

On opérant comme décrit pour l'exemple 1, mais en utilisant la 2-méthyl-N-éthyl-pipérazine au lieu de la 2,6-diméthyl-N-éthylpipérazine, on obtient le composé du titre.

Les composés selon l'invention ont fait l'objet d'études *in vivo.*

### Essai in vivo

Des rats mâles CrI CD BR (Charles River, Italie) pesant 150-175 g ont été logés dans une chambre à température (22±1°C), humidité (55 ± 10%) contrôlées et un cycle lumière-obscurité de 12 heures pendant au moins 7 jours avant leur utilisation. La nourriture et l'eau étaient disponibles *ad libitum.* L'alimentation a été retirée 18 heures avant le sacrifice des animaux. Les rats ont été sacrifiés par dislocation cervicale, l'estomac a été retiré chirurgicalement, ouvert le long de la courbure la plus faible, et placés dans une solution de Krebs (de composition (mM) : NaCl 118, 4; KCI 4,7 ; CaCl₂ 2,5 ; NaH₂PO₄ 3,7 ; MgSO₄ 1,2 ; NaHCO₃ 25 ; glucose 5,6). Le soin et le sacrifice des animaux ont été effectués selon le code éthique international de Sanofi-Aventis et les principes internationaux régissant le soin et le traitement des animaux de laboratoire (E.E.C. Directive 86/609, DJL358, 1, 12 décembre 1987). Des bandes d'environ 1 cm (5 mm de largeur) de fundus gastrique ont été coupées le long de l'axe longitudinal et suspendus dans 20 ml de bain rempli avec la solution de Krebs à 37°C et gazés avec un mélange gazeux de 95% O₂-5% CO₂. Les bandes ont été maintenues à une charge de repos de 1 g et après lavage de choline (précurseur de l'acétylcholine) 10 µM et 10 µM d'indométhacine (inhibiteur de prostaglandines synthétase) ont été ajoutés au milieu, pour réduire les contractions phasiques spontanées (Depoortere et al., Eur. J. Pharmacol. 515, 1-3, 160-168, 2003 ; Dass et al. Neurosciences 120, 443-453, 2003). Des contractions isotoniques ont été éveillées par stimulation par champ électrique. Deux électrodes de fil de platine ont été placées à la surface et au fond du bain d'organe et la stimulation par champ électrique a été réalisée par un stimulateur Power Lab (AD Instruments Pty Ltd. Castle Hill, Australie), couplé à un propulseur d'impulsion multiplex (Ugo Basile, Varese, Italie) (Fukuda et al., Scand. J. Gastroenterol. 12, 1209-1214, 2004). La stimulation supramaximale a été appliquée pour créer des contractions maximales (20 Hz, largeur de pulsation : 2 millisecondes ; 5 Volts ; trains de los toutes les 2 minutes, 150 mA). Ensuite, le courant a été réduit pour obtenir une stimulation submaximale (50% de réduction de la réponse contractile maximale). Les contractions ont été enregistrées par ordinateur avec un système d'enregistrement et d'analyse de données (Power Lab, Chart 5) relié à des transducteurs isotoniques (Ugo Basile, Varese, Italie) via des préamplificateurs (Octal Bridge Amp). Après stabilisation, des courbes cumulatives concentration-réponse de la ghréline (0,1 nM-1 µM) ont été tracées, avec et sans incubation (temps de contact : 30 mn) des molécules antagonistes. La stimulation par champ électrique supramaximale a été utilisée pour chaque bande comme référence (100 %) pour classer les réponses par substance test. La concentration agoniste produisant 50% d'effet maximal (EC50) a été calculée en utilisant un modèle logistique à quatre paramètres selon Ratkovsky et Reedy (Biometrics, 42, 575-582, 1986), avec ajustement par régression non linéaire en utilisant l'algorithme de Levenberg-Marquard dans le logiciel Everstat. Les valeurs pKB des antagonistes ont été calculées selon l'équation de Cheng-Prusoff (Kenakin et al., Competitive Antagonism, Pharmacologic Analysis of Drug-Receptor Interaction, 3e edition, 331-373, Philadelphie, New York ; Raven: Lippincott, 1997).

Les composés de formule (I) présentent une activité antagoniste du récepteur de la ghréline avec des CI₅₀ qui varient qui varient entre 5 10⁻⁸ M et 1 10⁻⁹M.

Par exemple, le composé de l'exemple n°2 a montré une CI₅₀ de 1,2 10⁻⁸M.

Les composés de formule (I) ont démontré des propriétés pharmacologiques intéressantes pour le développement d'un médicament, en particulier de médicaments destinés à prévenir ou à traiter toute pathologie où le récepteur de la ghréline est impliqué.

Ainsi, selon un autre de ses aspects, l'invention a pour objet des médicaments qui comprennent un composé de formule (I) ou un sel d'addition de ce dernier à un acide pharmaceutiquement acceptable.

Ainsi, les composés selon l'invention peuvent être utilisés, chez l'homme ou chez l'animal, dans le traitement ou la prévention de différentes affections ghréline dépendantes. Ainsi, les composés selon l'invention peuvent être utilisés comme agents anorexiques, pour réguler l'appétit, la prise de repas et leur fréquence, ainsi que, à long terme, le poids, notamment la prise de poids faisant suite aux régimes diététiques ou thérapeutiques. Les composés selon l'invention sont donc particulièrement utiles pour la prévention ou le traitement de l'obésité, des troubles de l'appétit, du diabète, la surcharge pondérale et/ou de leurs effets.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, ainsi qu'au moins un excipient pharmaceutiquement acceptable.

Les dits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains, pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 100 mg/kg, en une ou plusieurs prises. Par voie parentérale, elle peut atteindre 0.01 à 10 mg/Kg/jour.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

### Associations possibles

La présente invention concerne également les combinaisons d'un ou plusieurs composés(s) selon l'invention de formule générale (I) avec un ou plusieurs ingrédient(s) actif(s).

A titre d'ingrédient(s) actif(s) convenant auxdites combinaisons, on peut notamment citer les agents anti-obésité et antidiabétiques, ainsi que le rimonabant, la metformine ou les sulfonylurées.

Il est également décrit une méthode de traitement des pathologies ci-dessus indiquées qui comprend l'administration, à un patient, d'une dose efficace d'un composé selon l'invention, ou un de ses sels pharmaceutiquement acceptables.

La présente invention, selon un autre de ses aspects, concerne également un composé de formule (I), ou un de ses sels pharmaceutiquement acceptables, pour le traitement des pathologies ci-dessus indiquées.

## Revendications

1. Composé répondant à la formule (I) : 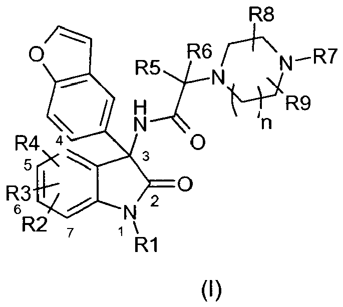 dans laquelle :
R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno(C1-3) alcoxy, aminocarbonyle, (C1-6) alkylaminocarbonyle, di(C1-6) alkylaminocarbonyle, aryle, aryloxy , hétéroaryle ;
le groupe aryle, aryloxy ou hétéroaryle pouvant éventuellement être substitué par un atome d'halogène, CN, OH ou un groupe (C1-6) alkyle , perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle , perhatogéno(C1-3) alkyle, (C1-6) alcoxy;
R5 et R6, identiques ou différents, représentent un atome d'hydrogène, un groupe (C1-6) alkyle ou R5 et R6 forment ensemble un cycle C3-C6 ;
R7 représente un groupe (C1-C6) alkyle ou un groupe (C2-6) alcényle ;
R8 et R9, situés sur l'une quelconque des positions disponibles du noyau de la pipérazine, représentent un atome d'hydrogène, un groupe (C1-C6) alkyle ou un groupe (C2-6) alcényle, étant entendu que au moins un des R8 et R9 est différent de H ;
ou deux des R7, R8 et R9 forment ensemble un cycle C3-C6 ;
étant entendu que R8 et R9 peuvent se trouver en position géminale sur le même atome de carbone ;
n représente 1 ou 2 ;
sous forme de base ou de sel d'addition à un acide.

2. Composé selon la revendication 1 tel que dans la formule générale (I) :
R1 représente un atome d'hydrogène ou un groupe (C1-6) alkyle, -C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle, perhalogéno(C1-3) alkyle, (C1-6) alcoxy, perhalogéno(C1-3) alcoxy, aminocarbonyle, (C1-6) alkylaminocarbonyle, di(C1-6) alkylaminocarbonyle, aryle, aryloxy, hétéroaryle, étant entendu que au moins un des R2, R3, R4 est différent de H ;
R5 et R6, identiques ou différents, représentent un atome d'hydrogène, un groupe (C1-6) alkyle ou R5 et R6 forment ensemble un cycle C3-C6;
R7 représente un groupe (C1-C6) alkyle;
R8 et R9, situés sur l'une quelconque des positions disponibles du noyau de la pipérazine, représentent un atome d'hydrogène ou un groupe (C1-C6) alkyle, étant entendu que au moins un des R8 et R9 est différent de H ;
ou deux des R7, R8 et R9 forment ensemble un cycle C3-C6 ;
n représente 1 ou 2 ;
sous forme de base ou de sel d'addition à un acide.

3. Composé selon la revendication 1 ou 2 tel que dans la formule générale (I) :
R1 représente un atome d'hydrogène ou un groupe -C(=O)(C1-6) alkyle, -C(=O)aryle, (C1-6)alkyle ;
R2, R3, R4, identiques ou différents, situés sur l'une quelconque des positions disponibles du noyau phényle, représentent indépendamment un atome d'hydrogène, un atome d'halogène, ou un groupe (C1-6) alkyle ou trifluorométhyle, étant entendu que au moins un des R2, R3, R4 est différent de H ;
R5 et R6, identiques ou différents, représentent un atome d'hydrogène, un groupe (C1-6) alkyle ;
R7 représente un groupe (C1-C6) alkyle;
R8 et R9, situés sur les positions 2 et 6 du noyau de la pipérazine, représentent un atome d'hydrogène ou un groupe (C1-C6) alkyle, étant entendu que au moins un des R8 et R9 est différent de H ;
ou deux des R7, R8 et R9 forment ensemble un cycle C3-C6;
n représente 1 ou 2 ;
sous forme de base ou de sel d'addition à un acide.

4. Composé selon l'une quelconque des revendications précédentes tel que dans la formule générale (I) :
R1 représente un atome d'hydrogène ou un groupe -C(=O)méthyle, -C(=O)phényle, méthyle ;
R2, R3, R4, identiques ou différents, situés sur l'une quelconque des positions disponibles du noyau phényle, représentent indépendamment un atome d'hydrogène, un atome d'halogène, ou un groupe méthyle ou trifluorométhyle, étant entendu que au moins un des R2, R3, R4 est différent de H ;
R5 et R6, identiques ou différents, représentent un atome d'hydrogène, un groupe (C1-6) alkyle ;
R7 représente un groupe méthyle ou éthyle;
R8 et R9, situés sur les positions 2 et 6 du noyau de la pipérazine, représentent un atome d'hydrogène ou un groupe méthyle, un groupe éthyle, étant entendu que au moins un des R8 et R9 est différent de H ;
ou deux des R7, R8 et R9 forment ensemble un cycle C3-C6 ;
n représente 1 ou 2 ;
sous forme de base ou de sel d'addition à un acide.

5. Composé selon l'une quelconque des revendications précédentes choisi parmi les composés suivants :
Composé n°1 : (+) N-[4,6-Dichloro-3-(benzofuran-5-yl)-2-oxo-2,3-dihydro-1 H-indol-3-yl]-2-(4-éthyl-pipérazin-1-yl)-acétamide ;
Composé n°2 : (+) N-[4,6-Dichloro-3-(benzofuran-5-yl)-2-oxo-2,3-dihydro-1H-indol-3-yl]-2-(4-éthyl-pipérazin-1-yl)-propionamide ;
sous forme de base ou de sel d'addition à un acide.

6. Procédé de préparation d'un composé de formule (I) selon l'une quelconque des revendications 1 à 5, **caractérisé en ce qu'**il comprend une étape consistant à faire réagir un composé de formule générale (V) : 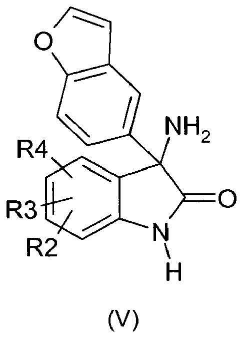 dans laquelle R2, R3 et R4 sont définis selon l'une quelconque des revendications 1 à 5.

7. Procédé selon la revendication 6 comprenant les étapes consistant à :
- faire réagir ledit composé de formule générale (V) avec un composé de formule générale (VI) : 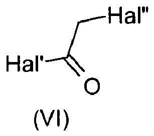 dans laquelle Hal' et Hal", identiques ou différents représentent indépendamment un atome d'halogène ;
- puis faire réagir le composé de formule générale (III) obtenu 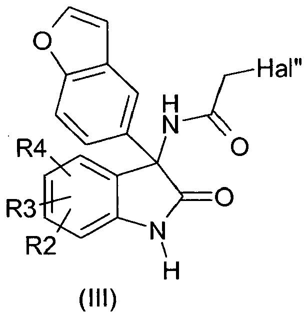 avec un composé de formule générale (IV) : 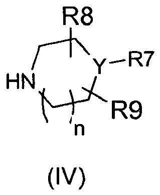 dans lesquelles R2, R3, R4, R7, R8, R9 et n sont tels que définis en formule générale (I), et Hal" représente un atome d'halogène ;
- suivie éventuellement de l'étape consistant à faire réagir le produit de formule (I) obtenu dans laquelle R1 est égal à H, avec un composé de formule (II) :
R1-Hal (II)
dans laquelle R1, différent de H est défini comme en formule générale (I) et Hal représente un atome d'halogène.

8. Procédé selon la revendication 6 comprenant l'étape consistant à faire réagir ledit composé de formule générale (V) avec un composé de formule générale (VII) : 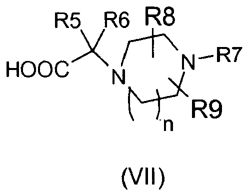 dans laquelle, R5, R6, R7, R8, R9 et n sont définis selon l'une quelconque des revendications 1 à 5,
suivie éventuellement de l'étape consistant à faire réagir le produit de formule (I) obtenu dans laquelle R1 est égal à H, avec un composé de formule (II) :
R1-Hal (II)
dans laquelle R1, différent de H est défini comme en formule générale (I) et Hal représente un atome d'halogène.

9. Procédé de préparation d'un composé de formule (I) selon l'une quelconque des revendications 1 à 5, **caractérisé en ce qu'**il comprend une étape consistant à faire réagir un composé de formule générale (XVII) : 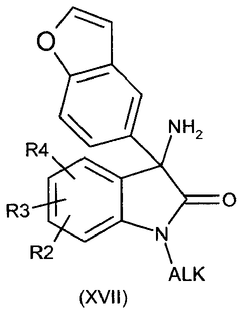 dans laquelle R2, R3 et R4 sont définis selon l'une quelconque des revendications 1 à 5 et ALK représente un groupe alkyle.

10. Procédé selon la revendication 9 comprenant les étapes consistant à :
- faire réagir ledit composé de formule générale (XVII) avec un composé de formule générale (VII) : 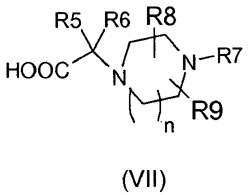 dans laquelle R5, R6, R7, R8, R9 et n sont tels que définis en formule générale (I).

11. Procédé selon l'une quelconque des revendications 6 à 10 comprenant l'étape ultérieure consistant à séparer le composé de formule générale (I) désiré.

12. Médicament, **caractérisé en ce qu'**il comprend un composé de formule (I) selon l'une quelconque des revendications 1 à 5, ou un sel d'addition de ce composé à un acide pharmaceutiquement acceptable.

13. Composition pharmaceutique, **caractérisée en ce qu'**elle comprend un composé de formule (I) selon l'une quelconque des revendications 1 à 5, ou un sel pharmaceutiquement acceptable.

14. Utilisation d'un composé selon l'une quelconque des revendications 1 à 5 pour la préparation d'un médicament destiné à la prévention ou au traitement de l'obésité, du diabète, des troubles de l'appétit et de la surcharge pondérale.

15. Composé selon l'une quelconque des revendications 1 à 5 pour la prévention ou le traitement de l'obésité, du diabète, des troubles de l'appétit et de la surcharge pondérale.

16. Combinaison comprenant un ou plusieurs composés(s) selon l'une quelconque des revendications 1 à 5 avec un ou plusieurs ingrédient(s) actif(s).

## Patentansprüche

1. Verbindung der Formel (I): 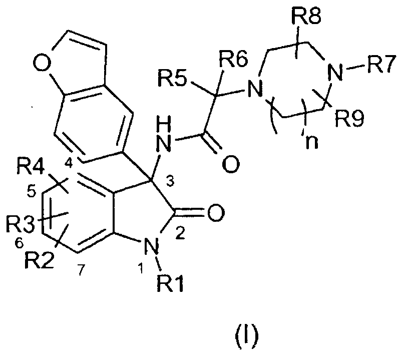 mit den folgenden Bedeutungen:
R1 bedeutet ein Wasserstoffatom oder eine (C1-6)-Alkylgruppe, -C(=O)(C1-6)-Alkylgruppe oder -C(=O)-Arylgruppe;
R2, R3, R4, die gleich oder verschieden sind und sich an einer beliebigen verfügbaren Position des Phenylrings befinden, bedeuten unabhängig ein Wasserstoffatom, ein Halogenatom, CN, OH, eine gegebenenfalls durch ein Halogenatom oder OH substituierte (C1-6)-Alkylgruppe; Perhalogen-(C1-3)-alkyl, (C1-6)-Alkoxy, Perhalogen-(C1-3)-alkoxy, Aminocarbonyl, (C1-6)-Alkylaminocarbonyl, Di-(C1-6)-Alkylaminocarbonyl, Aryl, Aryloxy, Heteroaryl;
wobei die Aryl-, Aryloxy- oder Heteroarylgruppe gegebenenfalls durch ein Halogenatom, CN, OH oder
eine (C1-6)-Alkylgruppe, Perhalogen(C1-3)-alkylgruppe oder (C1-6)-Alkoxygruppe substituiert sein kann; mit der Maßgabe, dass mindestens einer der Reste R2, R3, R4 nicht H bedeutet und dass die Aryl-, Aryloxy- oder Heteroarylgruppe gegebenenfalls durch ein Halogenatom, CN, OH oder
eine (C1-6)-Alkylgruppe, Perhalogen(C1-3)-alkylgruppe, (C1-6)-Alkoxygruppe substituiert sein kann;
R5 und R6, die gleich oder verschieden sind,
bedeuten ein Wasserstoffatom, eine (C1-6)-Alkylgruppe oder R5 und R6 bilden gemeinsam einen C3-C6-Zyklus;
R7 bedeutet eine (C1-6)-Alkylgruppe oder eine (C2-6)-Alkenylgruppe;
R8 und R9, die sich in einer beliebigen der verfügbaren Stellungen des Piperazinkerns befinden, bedeuten ein Wasserstoffatom, eine (C1-C6)-Alkylgruppe oder eine (C2-C6)-Alkenylgruppe,
wobei gilt, dass mindestens einer von R8 und R9 nicht H bedeutet;
oder zwei von R7, R8 und R9 bilden gemeinsam einen C3-C6-Zyklus;
wobei gilt, dass sich R8 und R9 in geminaler Stellung desselben Kohlenstoffatoms befinden können;
n bedeutet 1 oder 2;
in Basenform oder in Form eines Säureadditionssalzes.

2. Verbindung nach Anspruch 1, wobei die Symbole in der allgemeinen Formel (I) die folgenden Bedeutungen aufweisen:
R1 bedeutet ein Wasserstoffatom oder eine (C1-6)-Alkylgruppe, -C(=O)(C1-6)-Alkylgruppe, -C(=O)-Arylgruppe;
R2, R3, R4, die gleich oder verschieden sind und sich an einer beliebigen verfügbaren Position des Phenylrings befinden, bedeuten unabhängig ein Wasserstoffatom, ein Halogenatom, CN, OH, eine (C1-6)-Alkylgruppe; Perhalogen(C1-3)-alkylgruppe, (C1-6)-Alkoxygruppe, Perhalogen(C1-3)-alkoxygruppe, Aminocarbonylgruppe, (C1-6)-Alkylaminocarbonylgruppe, Di(C1-6)-Alkylaminocarbonylgruppe, Arylgruppe, Aryloxygruppe; Heteroaryl, mit der Maßgabe, dass mindestens einer der Reste R2, R3,
R4 nicht H bedeutet;
R5 und R6, die gleich oder verschieden sind,
bedeuten ein Wasserstoffatom, eine (C1-C6)-Alkylgruppe, oder R5 und R6 bilden gemeinsam einen C3-C6-Zyklus;
R7 bedeutet eine (C1-6)-Alkylgruppe;
R8 und R9, die sich in einer beliebigen der verfügbaren Stellungen des Piperazinkerns befinden, bedeuten ein Wasserstoffatom oder eine (C1-C6)-Alkylgruppe, wobei gilt, dass mindestens einer von R8 und R9 nicht H bedeutet;
oder zwei von R7, R8 und R9 bilden gemeinsam einen C3-C6-Zyklus;
n bedeutet 1 oder 2;
in Basenform oder in Form eines Säureadditionssalzes.

3. Verbindung nach Anspruch 1 oder 2, wobei die Symbole in der allgemeinen Formel (I) die folgenden Bedeutungen aufweisen:
R1 bedeutet ein Wasserstoffatom oder eine -C(=O)(C1-6)-Alkylgruppe, -C(=O)-Arylgruppe, (C1-6)-Alkylgruppe;
R2, R3, R4, die gleich oder verschieden sind und sich an einer beliebigen verfügbaren Position des Phenylrings befinden, bedeuten unabhängig ein Wasserstoffatom, ein Halogenatom, oder eine (C1-6)-Alkylgruppe oder Trifluormethylgruppe, mit der Maßgabe, dass mindestens einer der Reste R2, R3, R4 nicht H bedeutet;
R5 und R6, die gleich oder verschieden sind, bedeuten ein Wasserstoffatom, eine (C1-C6)-Alkylgruppe;
R7 bedeutet eine (C1-6)-Alkylgruppe;
R8 und R9, die sich in den Stellungen 2 und 6 des Piperazinkerns befinden, bedeuten ein Wasserstoffatom oder eine (C1-C6)-Alkylgruppe,
wobei gilt, dass mindestens einer von R8 und R9 nicht H bedeutet;
oder zwei von R7, R8 und R9 gemeinsam einen C3-C6-Zyklus bilden;
n bedeutet 1 oder 2;
in Basenform oder in Form eines Säureadditionssalzes.

4. Verbindung nach einem der vorhergehenden Ansprüche, wobei die Symbole in der allgemeinen Formel (I) die folgenden Bedeutungen aufweisen:
R1 bedeutet ein Wasserstoffatom oder eine -C(=O)-Methylgruppe, -C(=O)-Phenylgruppe, Methylgruppe;
R2, R3, R4, die gleich oder verschieden sind und sich an einer beliebigen verfügbaren Position des Phenylrings befinden, bedeuten unabhängig ein Wasserstoffatom, ein Halogenatom oder eine Methyl- oder Trifluormethylgruppe, mit der Maßgabe, dass mindestens einer der Reste R2, R3, R4 nicht H bedeutet;
R5 und R6, die gleich oder verschieden sind, bedeuten ein Wasserstoffatom, eine (C1-C6)-Alkylgruppe;
R7 bedeutet eine Methylgruppe, Ethylgruppe oder 2-Propylgruppe;
R8 und R9, die sich in den Stellungen 2 und 6 des Piperazinkerns befinden, bedeuten ein Wasserstoffatom oder eine Methylgruppe oder eine Ethylgruppe, wobei gilt, dass mindestens einer von R8 und R9 nicht H bedeutet;
oder zwei von R7, R8 und R9 bilden gemeinsam einen C3-C6-Zyklus;
n bedeutet 1 oder 2;
in Basenform oder in Form eines Säureadditionssalzes.

5. Verbindung nach einem der vorhergehenden Ansprüche, ausgewählt aus den folgenden Verbindungen:
Verbindung Nr. 1: (+) N-[4,6-Dichlor-3-(benzofuran-5-yl)-2-oxo-2,3-dihydro-1H-indol-3-yl]-2-(4-ethylpiperazin-1-yl)acetamid;
Verbindung Nr. 2: (+)-N-[4,6-Dichlor-3-(benzofuran-5-yl)-2-oxo-2,3-dihydro-1H-indol-3-yl]-2-(4-ethylpiperazin-1-yl)propionamid;
in Basenform oder in Form eines Säureadditionssalzes.

6. Verfahren zur Herstellung einer Verbindung der Formel (I) nach einem der Ansprüche 1 bis 5, **dadurch gekennzeichnet, dass** es den Schritt umfasst, der darin besteht, dass man eine Verbindung der allgemeinen Formel (V), 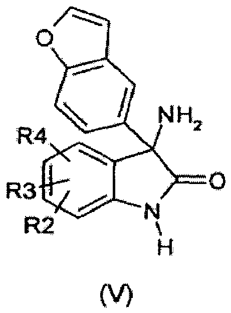 in der R2, R3 und R4 wie in einem der Ansprüche 1 bis 5 definiert sind,
umsetzt.

7. Verfahren nach Anspruch 6, umfassend die Schritte, die darin bestehen, dass man
- die Verbindung der allgemeinen Formel (V) mit einer Verbindung der allgemeinen Formel (VI), 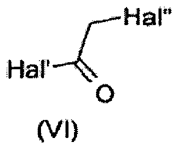 in der Hal' und Hal", die gleich oder verschieden sind, unabhängig ein Halogenatom bedeuten, umsetzt;
- dann die erhaltene Verbindung der allgemeinen Formel (III) 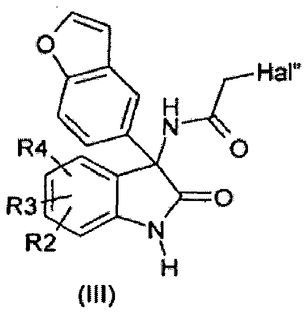 mit einer Verbindung der allgemeinen Formel (IV), 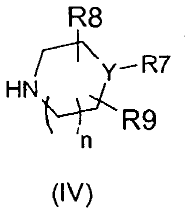 in der R2, R3, R4, R7, R8, R9 und n wie in der allgemeinen Formel (I) definiert sind und Hal" ein Halogenatom bedeutet,
umsetzt;
- gegebenenfalls gefolgt von dem Schritt, der darin besteht, dass man das erhaltene Produkt der Formel (I), in der R1 gleich H ist, mit einer Verbindung der Formel (II),
R1-Hal (II)
in der R1, das nicht H bedeutet, wie in der allgemeinen Formel (I) definiert ist und Hal ein Halogenatom bedeutet,
umsetzt.

8. Verfahren nach Anspruch 6, umfassend den Schritt, der darin besteht, dass man die Verbindung der allgemeinen Formel (V) mit einer Verbindung der allgemeinen Formel (VII), 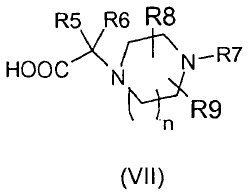 in der R5, R6, R7, R8, R9 und n wie in einem der Ansprüche 1 bis 5 definiert sind, umsetzt,
- gegebenenfalls gefolgt von dem Schritt, der darin besteht, dass man das erhaltene Produkt der Formel (I), in der R1 gleich H ist, mit einer Verbindung der Formel (II),
R1-Hal (II)
in der R1, das nicht H bedeutet, wie in der allgemeinen Formel (I) definiert ist und Hal ein Halogenatom bedeutet,
umsetzt.

9. Verfahren zur Herstellung einer Verbindung der Formel (I) nach einem der Ansprüche 1 bis 5, **dadurch gekennzeichnet, dass** es einen Schritt umfasst, der darin besteht, dass man eine Verbindung der allgemeinen Formel (XVII), 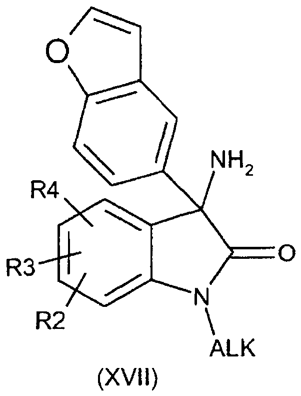 in der R2, R3 und R4 wie in einem der Ansprüche 1 bis 5 definiert sind und ALK eine Alkylgruppe bedeutet,
umsetzt.

10. Verfahren nach Anspruch 9, das die Schritte umfasst, die darin bestehen, dass man:
- die Verbindung der allgemeinen Formel (XVII) mit einer Verbindung der allgemeinen Formel (VII), 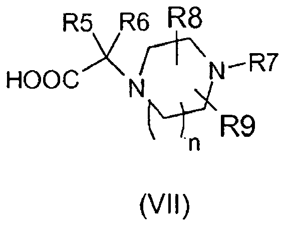 in der R5, R6, R7, R8, R9 und n wie in der allgemeinen Formel (I) definiert sind,
umsetzt.

11. Verfahren nach einem der Ansprüche 6 bis 10, das den weiteren Schritt umfasst, der darin besteht, dass man die gewünschte Verbindung der allgemeinen Formel (I) abtrennt.

12. Arzneimittel, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure umfasst.

13. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz umfasst.

14. Verwendung einer Verbindung nach einem der Ansprüche 1 bis 5 für die Herstellung eines Arzneimittels für die Vorbeugung oder Behandlung von Adipositas, Diabetes, Appetitstörungen und Übergewicht.

15. Verbindung nach einem der Ansprüche 1 bis 5 zur Vorbeugung oder Behandlung von Adipositas, Diabetes, Appetitstörungen und Übergewicht.

16. Kombination, die eine Verbindung oder mehrere Verbindungen nach einem der Ansprüche 1 bis 5 zusammen mit einem weiteren Wirkstoff bzw. weiteren Wirkstoffen umfasst.

## Claims

1. Compound corresponding to formula (I): 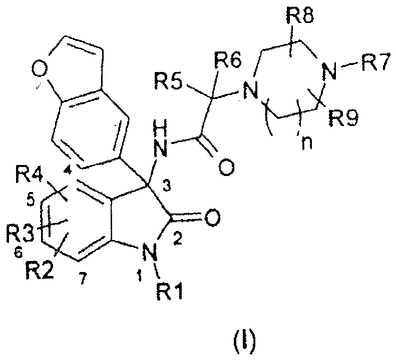 in which:
R1 represents a hydrogen atom or a (C1-6)alkyl, -C(=O)(C1-6)alkyl or -C(=O)aryl group;
R2, R3 and R4, which may be identical or different, located on any of the available positions of the phenyl nucleus, independently represent a hydrogen atom, a halogen atom, CN, OH, a (C1-6)alkyl group optionally substituted with a halogen atom or an OH; perhalo(C1-3)alkyl, (C1-6)alkoxy, perhalo(C1-3)alkoxy, aminocarbonyl, (C1-6)alkylaminocarbonyl, di(C1-6)alkylaminocarbonyl, aryl, aryloxy; heteroaryl;
the aryl, aryloxy or heteroaryl group possibly being optionally substituted with a halogen atom, CN, OH or a (C1-6)alkyl, perhalo(C1-3)alkyl or (C1-6)alkoxy group; it being understood that at least one from among R2, R3 and R4 is other than H and that the aryl, aryloxy or heteroaryl group may be optionally substituted with a halogen atom, CN, OH or a (C1-6)alkyl,
perhalo(C1-3)alkyl or (C1-6)alkoxy group;
R5 and R6, which may be identical or different, represent a hydrogen atom or a group (C1-6)alkyl or R5 and R6 together form a C3-C6 ring;
R7 represents a group (C1-C6)alkyl or a group (C2-6)alkenyl;
R8 and R9, which are located on any of the available positions of the piperazine nucleus, represent a hydrogen atom, a group (C1-C6)alkyl or a group (C2-6)alkenyl, it being understood that at least one from among R8 and R9 is other than H;
or two from among R7, R8 and R9 together form a C3-C6 ring;
it being understood that R8 and R9 may be in the geminal position on the same carbon atom;
n represents 1 or 2;
in the form of the base or of an acid-addition salt.

2. Compound according to Claim 1, such that, in the general formula (I):
R1 represents a hydrogen atom or a (C1-6)alkyl, -C(=O)(C1-6)alkyl or -C(=O)aryl group;
R2, R3 and R4, which may be identical or different, located on any of the available positions of the phenyl nucleus, independently represent a hydrogen atom, a halogen atom, CN, OH or a (C1-6)alkyl, perhalo(C1-3)alkyl, (C1-6)alkoxy, perhalo(C1-3)alkoxy, aminocarbonyl, (C1-6)alkylaminocarbonyl, di(C1-6)alkylaminocarbonyl, aryl, aryloxy or
heteroaryl group, it being understood that at least one from among R2, R3 and R4 is other than H;
R5 and R6, which may be identical or different, represent a hydrogen atom or a group (C1-6)alkyl or R5 and R6 together form a C3-C6 ring;
R7 represents a group (C1-C6)alkyl;
R8 and R9, which are located on any of the available positions of the piperazine nucleus, represent a hydrogen atom, a group (C1-C6)alkyl, it being understood that at least one from among R8 and R9 is other than H;
or two from among R7, R8 and R9 together form a C3-C6 ring;
n represents 1 or 2;
in the form of the base or of an acid-addition salt.

3. Compound according to Claim 1 or 2, such that, in the general formula (I):
R1 represents a hydrogen atom or a -C(=O)(C1-6)alkyl, -C(=O)aryl or (C1-6)alkyl group;
R2, R3 and R4, which may be identical or different, located on any of the available positions of the phenyl nucleus, independently represent a hydrogen atom, a halogen atom, or a (C1-6)alkyl or trifluoromethyl group, it being understood that at least one from among R2, R3 and R4 is other than H;
R5 and R6, which may be identical or different, represent a hydrogen atom or a group (C1-6)alkyl;
R7 represents a group (C1-C6)alkyl;
R8 and R9, which are located on positions 2 and 6 of the piperazine nucleus, represent a hydrogen atom, a group (C1-C6)alkyl, it being understood that at least one from among R8 and R9 is other than H;
or two from among R7, R8 and R9 together form a C3-C6 ring;
n represents 1 or 2;
in the form of the base or of an acid-addition salt.

4. Compound according to any one of the preceding claims, such that, in the general formula (I):
R1 represents a hydrogen atom or a -C(=O)methyl, -C(=O)phenyl or methyl group;
R2, R3 and R4, which may be identical or different, located on any of the available positions of the phenyl nucleus, independently represent a hydrogen atom, a halogen atom, or a methyl or trifluoromethyl group, it being understood that at least one from among R2, R3 and R4 is other than H;
R5 and R6, which may be identical or different, represent a hydrogen atom or a group (C1-6)alkyl;
R7 represents a methyl or ethyl group;
R8 and R9, which are located on positions 2 and 6 of the piperazine nucleus, represent a hydrogen atom or a methyl or ethyl group, it being understood that at least one from among R8 and R9 is other than H;
or two from among R7, R8 and R9 together form a C3-C6 ring;
n represents 1 or 2;
in the form of the base or of an acid-addition salt.

5. Compound according to any one of the preceding claims, chosen from the following compounds:
Compound No. 1: (+)-N-[4,6-dichloro-3-(benzofuran-5-yl)-2-oxo-2,3-dihydro-1H-indol-3-yl]-2-(4-ethylpiperazin-1-yl)acetamide;
Compound No. 2: (+)-N-[4,6-dichloro-3-(benzofuran-5-yl)-2-oxo-2,3-dihydro-1H-indol-3-yl]-2-(4-ethylpiperazin-1-yl)propionamide;
in the form of the base or of an acid-addition salt.

6. Process for preparing a compound of formula (I) according to any one of Claims 1 to 5, **characterized in that** it comprises a step that consists in reacting a compound of general formula (V): 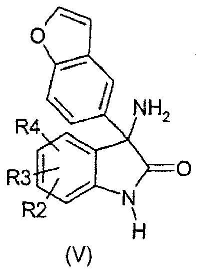 in which R2, R3 and R4 are as defined according to any one of Claims 1 to 5.

7. Process according to Claim 6, comprising the steps consisting in:
- reacting the said compound of general formula (V) with a compound of general formula (VI): 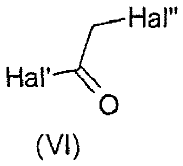 in which Hal' and Hal", which may be identical or different, independently represent a halogen atom;
- and then in reacting the compound of general formula (III) obtained 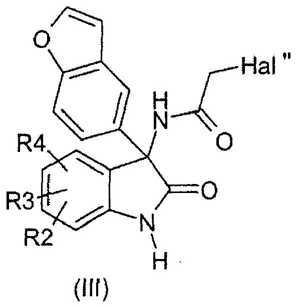 with a compound of general formula (IV): 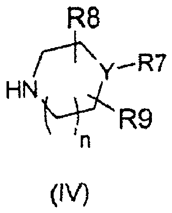 in which R2, R3, R4, R7, R8, R9 and n are defined as in the general formula (I) and Hal" represents a halogen atom;
- optionally followed by the step that consists in reacting the product of formula (I) obtained, in which R1 is equal to H, with a compound of formula (II):
R1-Hal (II)
in which R1, which is other than H, is defined as in the general formula (I) and Hal represents a halogen atom.

8. Process according to Claim 6, comprising the step that consists in reacting the said compound of general formula (V) with a compound of general formula (VII): 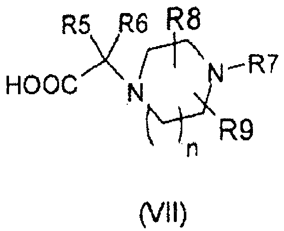 in which R5, R6, R7, R8, R9 and n are as defined according to any one of Claims 1 to 5;
optionally followed by the step that consists in reacting the product of formula (I) obtained, in which R1 is equal to H, with a compound of formula (II):
R1-Hal (II)
in which R1, which is other than H, is defined as in the general formula (I) and Hal represents a halogen atom.

9. Process for preparing a compound of formula (I) according to any one of Claims 1 to 5, **characterized in that** it comprises a step that consists in reacting a compound of general formula (XVII): 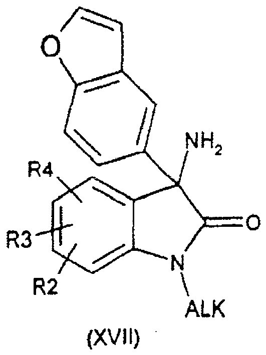 in which R2, R3 and R4 are defined according to any one of Claims 1 to 5 and ALK represents an alkyl group.

10. Process according to Claim 9, comprising the step that consists in:
- reacting the said compound of general formula (XVII) with a compound of general formula (VII): 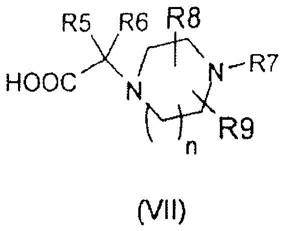 in which R5, R6, R7, R8, R9 and n are as defined in general formula (I).

11. Process according to any one of Claims 6 to 10, comprising the subsequent step that consists in separating out the desired compound of general formula (I).

12. Medicament, **characterized in that** it comprises a compound of formula (I) according to any one of Claims 1 to 5, or an addition salt of this compound with a pharmaceutically acceptable acid.

13. Pharmaceutical composition, **characterized in that** it comprises a compound of formula (I) according to any one of Claims 1 to 5, or a pharmaceutically acceptable salt.

14. Use of a compound according to any one of Claims 1 to 5 for the preparation of a medicament for preventing or treating obesity, diabetes, appetite disorders and excess weight.

15. Compound according to any one of Claims 1 to 5, for preventing or treating obesity, diabetes, appetite disorders and excess weight.

16. Combination comprising one or more compounds according to any one of Claims 1 to 5 with one or more active ingredient(s).
